Europäisches Patentamt

European Patent Office

Office européen des brevets

⊙ Publication number: **0 296 324**
**A2**

# EUROPEAN PATENT APPLICATION

Application number: 88106055.2

Int. Cl.⁴ **A61F 13/00 , A61F 13/10**

Date of filing: **13.11.85**

Priority: **21.11.84 DK 5528/84**

Date of publication of application:
**28.12.88 Bulletin 88/52**

Publication number of the earlier application in
accordance with Art.76 EPC: **0 185 197**

Designated Contracting States:
**DE FR GB IT NL SE**

Applicant: **Mölnlycke AB**

**S-405 03 Göteborg(SE)**

Applicant: **Tytex A/S**
**Industrivej 21**
**DK-7430 Ikast(DK)**

Inventor: **Kristensen, Johannes Nyvang**
**Laesogade 3**
**DK-7430 Ikast(DK)**
Inventor: **Thygesen, Eskild Geord**
**Broholmvej 14 Tulstrup**
**DK-7430 Ikast(DK)**
Inventor: **Grindsted, Erik**
**Frederiksberg Alle 19A 4. th**
**DK-1820 Frederiksberg C(DK)**
Inventor: **Jundell, Malte**
**Bokvägen 8**
**S-435 00 Mölnlycke(SV)**

Representative: **Patentanwälte Leinweber &**
**Zimmermann**
**Rosental 7/II Aufg.**
**D-8000 München 2(DE)**

## A fixation bandage for holding a dressing in position on the hand of a person.

A fixation bandage made from elastic material and adapted to hold a dressing such as a compress or the like firmly in a position on a hand of a person, is mainly formed as a hose in one piece preferably by knitting or crocheting.

The hose comprises a holding portion (7") adapted to receive a hand of a person, a fastening portion (5") and at least one gap (3). The fastening portion (5") forms a cuff, a collar or the like, which is intended to embrace a hand joint of a person.

The fixation bandage is so shaped and has an elasticity such that both the holding portion (7") and the fastening portion (5") can be slipped over the hand in such a manner as to have its fastening portion placed around the joint.

By means of the flexible and elastic fixation bandage thus provided the firm holding of the dressing in position is ensured, even in the case of extreme movements of the hand. Moreover, the fixation bandage is easy to apply whether this is done by the injured person or by a person assisting him in the application of the fixation bandage.

FIG. 2

# A FIXATION BANDAGE FOR HOLDING A DRESSING IN POSITION ON THE HAND OF A PERSON

The present invention relates to a fixation bandage consisting of elastic material and intended to hold a wound dressing, such as a compress, firmly in position on a peripheral body portion of a person. said fixation bandage being preferably made by knitting or crocheting and being shaped mainly as a hose or said hose or flexible tube comprising a holding portion for receiving the body portion considered, at least one fastening portion and at least one gap, said fastening portion forming a cuff, collar, loop or the like for embracing a joint adjacent to the body portion in question of a person, the fixation bandage being so shaped and having an elasticity such that both the holding portion and the fastening portion can be slipped over the body portion considered in such a manner as to have its fastening portion placed around the adjacent joint.

A fixation bandage of this type is disclosed in US-A-3 279 465. This prior fixation bandage is adapted to be placed on those parts of the human body, like the trunk and the abdomen. The bandage has the shape of a T-shirt and is made of a knitted fabric comprising inelastic yarns and elastic yarns which are arranged in a zig-zag configuration.

It is known to hold wound dressings in position by means of conventional bandages. particularly gauze bandages. However, this has the disadvantage that the correct holding of the wound dressing depends very much on the tightness with which the bandage has been applied so that there is a risk that the holding of the wound dressing may become either too loose or too tight.

It is also known to hold wound dressings in position by means of elastic tubular meshed fabrics. which are intended to provide an improved holding of the dressing because they can expand and contract in all direction. Thereby the above mentioned drawback in respect of tightness of the bandage is eliminated. However. these tubular meshed fabrics cannot be used in connection with all types of dressings.

In the case of injuries to the back or the palm of the hand. compresses or the like are fastened by means of a gauze bandage which is wound around the hand. Since the hand is usually moved to a considerable extent, it is difficult to apply a bandage in such a manner that it both permits certain movements of the hand and holds the compresses in position in a stable manner.

It is the object of the present invention to remedy the drawbacks mentioned above by providing a fixation bandage which is flexible and elastic and ensures the maintenance in position of the dressing on the head even in the case of extreme movements thereof. and which is easy to apply, whether it is done by the injured person himself or by a person assisting him for the purpose.

To achieve this object the invention is **characterized in** that the hose is wholly open at one end (2″) in the fasteining portion (5″), which is formed as a cuff for placing around the wrist, that the hose is provided at its opposite end (1″) with a transverse connection (19) adapted. upon slipping the hose over the hand, to be engaged between two fingers, and that the gap (3″) is provided substantially at the middle of the hose and is adapted to form a free opening for the thumb.

Further developments of the invention are set forth in the depending claims.

The invention will now be described in more detail with reference to an exemplifying, non-limiting embodiment thereof illustrated in the accompanying drawings, in which

Fig. 1 shows a fixation bandage adapted to be applied to a hand, and

Figs 2 is an illustration of the fixation bandage of Fig. 1 as applied to a hand.

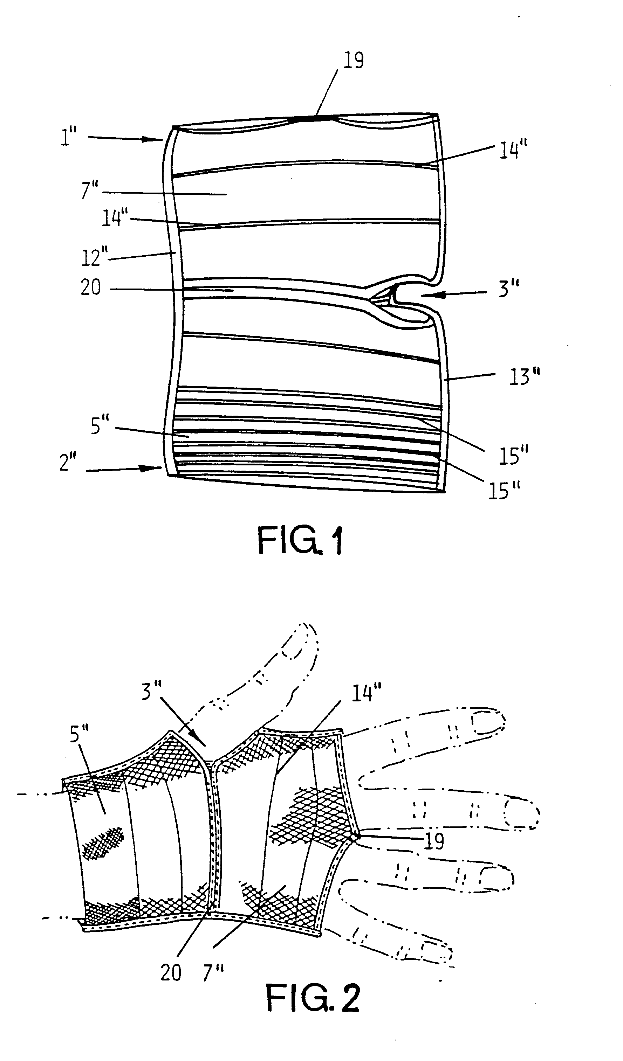

Fig. 1 shows a fixation bandage which is adapted to be placed on a hand. The fixation bandage is produced from two pieces knitted from elastic material and interconnected along the marginal edges 12″ and 13″. The hose-like unit thereby formed has a cross connection 19 in the middle of one end 1″.

At the other end 2″ a tightly knitted portion is provided including elastic re-inforcement threads 15″. This tightly knitted portion is intended to function as a cuff 5″.

In the holding portion 7″, which is knitted more open as compared with the cuff, a gap 3″ is provided which in the use of the fixation bandage is intended to serve as a free opening for the thumb. Directly flush with the gap 3″ the hose is provided with a relatively strong elastic 20, and elastic re-inforcement threads 14″ are provided at regular intervls transversely of the holding portion 7″.

Fig. 2 shows the fixation bandage of Fig.1 as applied to a hand. The holding portion 7″ is held in position by means of the cuff 5″, the cross connection 19 engaging between two fingers and the gap 3″ engaged by the thumb of the user.

## Claims

1. A fixation bandage consisting of elastic material and intended to hold a wound dressing, such as a compress. firmly in position on a peripheral body portion of a person. said fixation bandage being preferably made by knitting or crocheting and being shaped mainly as a hose or flexible tube in one piece, said hose or flexible tube comprising a holding portion (7) for receiving the body portion considered, at least one fastening portion (5) and at least one gap (3), said fastening portion forming a cuff, collar, loop or the like for embracing a joint adjacent to the body portion in question of a person, the fixation bandage being so shaped and having an elasticity such that both the holding portion (7) and the fastening portion (5) can be slipped over the body portion considered in such a manner as to have its fastening portion placed around the adjacent joint, **characterized in** that the hose is wholly open at one end (2") in the fastening portion (5"), which is formed as a cuff for placing around the wrist ,that the hose is provided at its opposite end (1") with a transverse connection (19) adapted, upon slipping the hose over the hand, to be engaged between two fingers, and that, the gap (3") is provided substantially at the middle of the hose and is adapted to form a free opening for the thumb.

2. A fixation bandage as in claim 1, **characterized in** that the hose is made with different types of knitting (a-b) distributed over the length of the hose.

3. A fixation bandage as in any of the beforegoing claims, **characterized in** that the hose is provided with a colour code (11) to indicate size.

4. A fixation bandage as in any of the beforegoing claims, **characterized in** that the hose is made from two knitted or crocheted webs which are interconnected at their edges (12, 13) to form the hose.

19

1"

14"

7"

14"

12"

20

3"

13"

5"

2"

15"

15"

## FIG. 1

3"

14"

5"

19

20  7"

## FIG. 2